(19) Europäisches Patentamt

European Patent Office

Office européen des brevets

(11) Veröffentlichungsnummer: **0 268 100 B1**

(12) **EUROPÄISCHE PATENTSCHRIFT**

(45) Veröffentlichungstag der Patentschrift: **12.06.91**

(51) Int. Cl.⁵: **A61K 31/275**

(21) Anmeldenummer: **87115417.5**

(22) Anmeldetag: **21.10.87**

Die Akte enthält technische Angaben, die nach dem Eingang der Anmeldung eingereicht wurden und die nicht in dieser Patentschrift enthalten sind.

(54) **Wirkstoffe zur Anwendung bei der Behandlung von Tumoren.**

(30) Priorität: **22.10.86 DE 3635930**

(43) Veröffentlichungstag der Anmeldung:
**25.05.88 Patentblatt 88/21**

(45) Bekanntmachung des Hinweises auf die Patenterteilung:
**12.06.91 Patentblatt 91/24**

(84) Benannte Vertragsstaaten:
**AT BE CH DE FR GB IT LI NL SE**

(56) Entgegenhaltungen:
**EP-A- 0 147 707**
**EP-A- 0 159 678**
**DE-A- 2 059 985**

**ARZNEIMITTEL FORSCHUNG, DRUG RESEARCH, Band 35, Nr. 11, November 1985, Seiten 1717-1719, Editio Cantor, Aulendorf, DE; W. SCHEID et al.: "Synergistic enhancement of the bleomycin and peplomycin induced mitotic index reduction by verapamil"**

**CHEMICAL ABSTRACTS, Band 99, Nr. 9, 29. August 1983, Seite 68, Zusammenfassung Nr. 64298e, Columbus, Ohio, US; & JP-A-58 83 624 (EISAI CO., LTD) 19-05-1983 (Kat. D)**

(73) Patentinhaber: **BASF Aktiengesellschaft**
**Carl-Bosch-Strasse 38**
**W-6700 Ludwigshafen(DE)**

(72) Erfinder: **Kretzschmar, Rolf, Prof. Dr.**
**Oberer Bergelweg 4**
**W-6718 Gruenstadt(DE)**
Erfinder: **Schlick, Erich, Dr.**
**Rudolph-Wihr-Strasse 4 1/2**
**W-6708 Neuhofen(DE)**
Erfinder: **Eichelbaum, Michel, Prof. Dr.**
**Am Ring 32**
**W-7140 Ludwigsburg(DE)**

CHEMICAL ABSTRACTS, Band 105, Nr. 19, 10.
November 1986, Seite 41, Zusammenfassung
nr. 164690n, Columbus, Ohio, US; M.J. CUR-
TIS et al.: "The calcium antagonist potency
ratio of the optical enantiomers of verapamil
in a variety of preparations", & PROC. WEST.
PHARMACOL. SOC. 1986, 29, 295-7

CHEMICAL ABSTRACTS, Band 106, Nr. 17, 27.
April 1987, Seite 36, Zusammenfassung Nr.
131442a, Columbus, Ohio, US; B.A. MACLE-
OD et al.: "An improved pithed rat preparation: the actions of the optical enantiomers
of verapamil", & ASIA PAC. J. PHARMACOL
1986, 1(2), 73-8

**Beschreibung**

In der DE-B- 1 154 810 sind basisch substituierte Phenylacetonitrile beschrieben. Aus dieser Verbindungsklasse haben sich aufgrund ihrer calciumantagonistischen Wirkung in der Therapie der koronaren Herzkrankheit und des Bluthochdrucks das Verapamil und das Gallopamil bewährt. Beide Verbindungen werden in der Therapie in racemischer Form eingesetzt. In der DE-A- 2 059 923 sind die linksdrehenden Antipoden von Verapamil und Gallopamil beschrieben. Beide Verbindungen sind dem Racemat in der Coronarwirksamkeit deutlich überlegen. In der DE-C- 20 59 985 sind die rechtsdrehenden Antipoden von Verapamil und Gallopamil offenbart. Beide rechtsdrehende Verbindungen sind dem Racemat bezüglich der Coronarwirksamkeit unterlegen.

In der EP-A- 147 707 sind die Antipoden von Emopamil und ihre Verwendung zur protektiven Behandlung von hypoxischen Gewebsschädigungen beschrieben. Beide Enantiomere zeigen dosisabhängig protektive Wirkung. Die Wirkdosis des linksdrehenden Antipoden liegt um den Faktor 8 bis 10 niedriger als die des rechtsdrehenden Antipoden.

Die deutliche Überlegenheit der linksdrehenden Antipoden von Verapamil, Gallopamil und Devapamil hinsichtlich der cardialen Wirkung ist auch der Arbeit von H. Nawrath und M. Raschack (Cell. Calcium 5, 316 (1984)) zu entnehmen. Die linksdrehenden Antipoden sind in ihrer calciumantagonistischen Wirkung bis zum Faktor 200 den rechtsdrehenden Antipoden überlegen. In der negativen inotropen Wirkung wurde ein Abstand bis zum Faktor 90 gefunden.

Weiterhin ist in der JP-A- 83 624/1983 die Verwendung von racemischem Verapamil zur Wirkverstärkung von Antitumormitteln beschrieben. Von A.B. Benson u.a. (Cancer Treat. Rep. 69, 795 (1985)) ist jedoch beschrieben, daß die Kombination von Verapamil mit Antitumormitteln nur beschränkt therapeutisch eingesetzt werden kann, da die kardiale Eigenwirkung von Verapamil eine erforderliche höhere Dosierung ausschließt.

Es ist weiter bekannt, daß praktisch alle Cancerostatica unerwünschte Nebenwirkungen besitzen oder daß ihre Antitumorwirkung mit der Zeit wegen Resistenzerscheinungen nachläßt.

Überraschenderweise wurde nun gefunden, daß sich die beiden enantiomeren Formen von Verapamil, Gallopamil, Devapamil und Emopamil hinsitich ihrer zytotoxizitätssteigernden Wirkung von Antitumormitteln nicht unterscheiden. Da die kardiale Wirkung der Racemate überwiegend durch die linksdrehenden Antipoden zustande kommt, bietet die Verwendung der rechtsdrehenden Enantiomeren die Möglichkeit, in ausreichend hohen Dosen zu applizieren bei gleichzeitiger Minimierung der kardialen Eigenwirkung. Dies stellt in Bezug auf die cardiale Eigenwirkung eine wesentliche Verbesserung des therapeutischen Indexes dar.

Erst durch die Verwendung der rechtsdrehenden Antipoden kann eine ausreichende Wirkungsverstärkung von Antitumormitteln erzielt werden.

Die vorliegende Erfindung betrifft Erzeugnisse enthaltend einerseits (+)-Verapamil, (+)-Gallopamil, (+)-Devapamil und/oder (+)-Emopamil und andererseits ein Cancerostaticum als Kombinationspräparat zur gleichzeitigen, getrennten oder zeitlich abgestuften Anwendung in der cytostatischen Therapie.

Verapamil ist 5-[(3,4-Dimethoxyphenethyl)-methylamino]-2-isopropyl-2-(3,4-dimethoxyphenyl)-valeronitril,

Gallopamil ist 5-[(3,4-Dimethoxyphenethyl)-methylamino]-2-isopropyl-2-(3,4,5-trimethoxyphenyl)-valeronitril,

Devapamil ist 5-[(3-Methoxyphenethyl)-methylamino]-2-isopropyl-2-(3,4-dimethoxyphenyl -valeronitril,

Emopamil ist 5-[(Phenethyl)methylamino]-2-isopropyl-2-phenyl-valeronitril,

Als Cancerostatica kommen insbesondere in Betracht:

a) Antibiotica wie Actinomycin D, Doxorubicin (Adriamycin), Daunorubicin, Mithramycin und Bleomycin sowie andere interchalatorisch wirkende Substanzen, wie Amonafide und Mitonafide,

b) Alkaloide wie Vincristin, Vinblastin, Vindesin, Etoposid und Teniposid,

c) alkylierend wirkende Substanzen, wie Cyclophosphamid, Nitrosoharnstoffe und Cisplatin und

d) Antimetabolite wie Methotrexat, 5-Fluoruracil und dessen Analoge, 6-Mercaptopurin, 6-Thioguanin und Cytarabin.

Die oben genannten Substanzen können gewünschtenfalls in Form ihrer Salze mit physiologischen Säuren bzw. Basen vorliegen. Als physiologisch verträgliche Säuren kommen vorzugsweise in Betracht: Salzsäure, Schwefelsäure, Phosphorsäure, Essigsäure, Zitronensäure, Malonsäure, Salicylsäure, Maleinsäure, Fumarsäure, Bernsteinsäure, Ascorbinsäure, Äpfelsäure, Methansulfonsäure, Milchsäure, Gluconsäure, Glucuronsäure, Amidosulfonsäure, Benzoesäure, Weinsäure.

Als physiologisch verträgliche Basen eignen sich insbesondere Ammoniak, Alkalimetallhydroxide - insbesondere des Natriums, Kaliums und Lithiums-, Erdalkalimetallhydroxide - besonders des Calciums und Magnesiums-, sowie organische Basen wie niedere Alkylamine - z.B. Methylamin oder Ethylamin-, Cyclohexylamin, substituierte niedere Alkylamine - z.B. Diethanolamin, Triethanolamin, Tris-(hyroxymethyl)-aminomethan -, Piperidin oder Morpholin.

Durch die neuen Kombinationen wird nicht nur das Wachstum von Krebszellen, sondern auch die Bildung von Metastasen verhindert oder stark eingeschränkt.

(+)-Verapamil, (+)-Gallopamil, (+)-Devapamil und/oder (+)-Emopamil (nachfolgend "Antipoden" bezeichnet) können zusammen oder getrennt mit den Cancerostatica verabfolgt werden. Bevorzugt ist jedoch die getrennte, vorherige Applikation sowie die getrennte vorherige Applikation mit anschließender gleichzeitiger Applikation von Antipode plus Cancerostaticum. Die Antipoden werden in der Regel oral appliziert, während die Cancerostatica oral oder parenteral (z.B. i.v., i.p.) gegeben werden.

Das Verhältnis von Antipoden zu Cancerostaticum hängt von der zu behandelnden Krebsart, dem Krankheitszustand des Patienten und dem verwendeten Cancerostaticum ab. In der Regel beträgt das Verhältnis etwa 1:1 bis 500:1. Dabei werden die Antipoden in der Regel in einer Menge von 200 bis 1000 mg pro Patient und Tag bei oraler Gabe und 200 bis 300 mg bei intravenöser bzw. 200 bis 500 mg bei intraperitonealer Gabe pro Patient und Tag angewendet. Die Cancerostatica werden in der Menge appliziert, die auch für die alleinige Gabe dieser Substanzen vorgesehen ist und die z.B. aus der "Rote Liste 1986" bzw. den darin erwähnten wissenschaftichen Prospekten entnommen werden kann.

Die Substanzen können in Form von Tabletten, Kapseln oder Dragees zur oralen Applikation oder als Injektionslösung zur parenteralen (i.v., i.p., i.m.) Applikation vorliegen. Lösungen können auch infundiert werden. Die Herstellung der Applikationsformen geschieht in bekannter Weise nach üblichen Methoden.

Herstellungsbeispiele

1. 500 mg (+)-Verapamil werden in 250 ml physiologischer Kochsalzlösung gelöst, sterilisiert und unter sterilen Bedingungen in eine Infusionsflasche gefüllt. Diese Lösung kann mit und/oder vor der Gabe eines Cancerostaticums appliziert werden.

2. 10 Ampullen mit je 1,5 mg Mithramycin "Pfizer" (vgl. Rote Liste 1985, Nr. 85 038) und eine Durchdrückpackung mit 10 Oblongtabletten mit (+)-Verapamil-Hydrochlorid wurden zusammen in eine Schachtel gepackt. Die Oblongtabletten waren in üblicher Weise hergestellt worden und enthielten pro Tablette 500 mg (+)-Verapamil, 120 mg Lactose, 60 mg Cellulose, 3 mg Magnesiumstearat, 50 mg Maisstärke und 15 mg Polyvinylpyrrolidon.

Die Fähigkeit der beiden enantiomeren Formen von Verapamil, Gallopamil, Devapamil und Emopamil, die zytotoxischen Eigenschaften von Antitumormitteln zu verstärken, wurde folgendermaßen gezeigt:

Als Testsystem diente eine murine Zytostatika-sensitive Zellinie sowie ein davon abgeleiteter resistenter Klon. Letzterer wurde durch Mutagenisierung mit Nitrosoguanidin und anschließende mehrfache Selektionierung gegen Adriamycin gewonnen.

$5 \times 10^3$ frisch trypsinisierte, exponentiell wachsende Zellen wurden in 96-Loch-Platten in einem Volumen von je $100\mu l$ komplettem Wachstumsmedium (RPMI 1640 mit 10 % FCS und 50 $\mu g/ml$ Gentamycin (Medium und Substanzen: Flow Laboratories, Meckenheim, FRG) ausplattiert und in einer wasserdampf-gesättigten Atmosphäre mit 5 % $CO_2$ bei 37°C inkubiert. Die Substanzzugabe erfolgte nach 24 h. Dabei wurden die Adriamycin-Konzentrationen von $10^{-6}M$ und $10^{-7}M$ allein und gegen verschiedene Konzentrationen ($10^{-5}M$-$10^{-6}M$) der $Ca^{2+}$-Antagonisten Verapamil, Gallopamil, Devapamil und Emopamil getestet. Als zusätzliche Kontrollen dienten Zellen ohne Wirksubstanz bzw. Zellen nur unter Einfluß der beschriebenen $Ca^{2+}$-Antagonisten. Das Endvolumen pro Loch war jeweils $200\mu l$. Nach einer weiteren Inkubation von 72 h bei den oben angegebenen Bedingungen wurden die überlebenden Zellen mit einer Kristallviolettlösung (15 g Kristallviolett, 7 g NaCl, 646 ml Ethanol, 172,8 ml 37 %iges Formaldehyd auf 2 l mit $H_2O$ aufgefüllt) angefärbt. Dazu wurden nach dem Ausschlagen des Kulturmediums die Zellen für 20 min mit $50\mu l$ der Färbelösung bei Raumtemperatur versetzt. Die Kulturplatten wurden danach mit Wasser gewaschen, um ungebundene Farbstoffanteile zu entfernen. Die gefärbten Zellen wurden durch Zugabe von $100\mu l$ Färbelösung (50 % Ethanol, 0,1 % Essigsäure) lysiert und bei 540 nm mit Hilfe eines Titertek Multiscan MCC/340 (Flow Laboratories, Meckenheim) photometrisch ausgewertet.

Die folgende Tabelle zeigte die so erhaltenen Werte. Darin bedeutet X den negativen Logarithmus der Konzentration C, gemessen in Mol/l. Y bedeutet den prozentualen Anteil getöteter Zellen des resistenten Klons, bezogen auf die unbehandelte Zellkontrolle.

| Substanz | Y nach Gabe von Adriamycin | | Y nach Gabe von Calciumantagonist allein |
|---|---|---|---|
| | X = 6 | X = 7 | X = 5 |
| (+)-Verapamil X = 5 | 71 | 51 | 12 |
| (+)-Verapamil X = 6 | 49 | 22 | |
| unbehandelte Zellkontrolle | 15 | 9 | |
| (-)-Verapamil X = 5 | 75 | 47 | 10 |
| (-)-Verapamil X = 6 | 47 | 19 | |
| unbehandelte Zellkontrolle | 13 | 6 | |
| (+)-Devapamil X = 5 | 66 | 41 | 11 |
| (+)-Devapamil X = 6 | 47 | 20 | |
| unbehandelte Zellkontrolle | 13 | 0 | |
| (-)-Devapamil X = 5 | 62 | 42 | 9 |
| (-)-Devapamil X = 6 | 41 | 5 | |
| unbehandelte Zellkontrolle | 7 | 0 | |
| (+)-Gallopamil X = 5 | 67 | 51 | 16 |
| (+)-Gallopamil X = 6 | 49 | 21 | |
| unbehandelte Zellkontrolle | 17 | 8 | |
| (-)-Gallopamil X = 5 | 64 | 48 | 18 |
| (-)-Gallopamil X = 6 | 43 | 15 | |
| unbehandelte Zellkontrolle | 16 | 8 | |
| (+)-Emopamil X = 5 | 51 | 45 | 30 |
| (+)-Emopamil X = 6 | 26 | 14 | |
| unbehandelte Zellkontrolle | 4 | 0 | |
| (-)-Emopamil X = 5 | 46 | 38 | 25 |
| (-)-Emopamil X = 6 | 24 | 13 | |
| unbehandelte Zellkontrolle | 8 | 3 | |

**Ansprüche**

1. Erzeugnisse enthaltend einerseits (+)-Verapamil, (+)-Gallopamil, (+)-Devapamil und/oder (+)-Emopamil und andererseits ein Cancerostaticum als Kombinationspräparat zur gleichzeitigen, getrennten oder zeitlich abgestuften Anwendung in der cytostatischen Therapie.

2. Erzeugnisse gemäß Anspruch 1, dadurch gekennzeichnet, daß sie (+)-Verapamil, (+)-Gallopamil, (+)-Devapamil und/oder (+)-Emopamil und andererseits ein Cancerostaticum im Verhältnis von etwa 1:1 bis 500:1 enthalten.

3. Verwendung von (+)-Verapamil, (+)-Gallopamil, (+)-Davapamil und/oder (+)-Emopamil zur Herstellung eines Arzneimittels zur Wirkungsverstärkung von Cancerostatica.

**Claims**

1. A product containing, on the one hand, (+)-verapamil, (+)-gallopamil, (+)-devapamil or (+)-emopamil and, on the other hand, a cancerostatic as a combination preparation for use simultaneously, separately or in stages in cytostatic therapy.

2. A product as claimed in claim 1, which contains (+)-verapamil, (+)-gallopamil, (+)-devapamil or (+)-emopamil and, on the other hand, a cancerostatic in a ratio of from about 1:1 to 500:1.

3. Use of (+)-verapamil, (+)-gallopamil, (+)-devapamil or (+)-emopamil for the preparation of a drug for reinforcing the action of cancerostatics.


**Revendications**

1. Produits contenant, d'une part, (+)-vérapamile, (+)-gallopamile, (+)-devapamile et/ou (+)-émopamile et, d'autre part, un cancérostatique, comme préparation de combinaison pour l'utilisation simultanée, séparée ou échelonnée dans le temps en thérapie cytostatique

2. Produits selon la revendication 1, caractérisé par le fait qu'ils contiennent (+)-vérapamile, (+)-gallopamile, (+)-devapamile et/ou (+)-émopamile et par ailleurs un cancérostatique en proportions d'environ 1/1 à 500/1.

3. Utilisation de (+)-vérapamile, (+)-gallopamile, (+)-devapamile et/ou (+)-émopamile pour la préparation d'un médicament pour l'augmentation d'efficacité des cancérostatiques.